# EUROPEAN PATENT SPECIFICATION

(11) **EP 1 485 084 B1**
(45) Date of publication and mention of the grant of the patent: **05.04.2006**
(21) Application number: 03744341.3
(22) Date of filing: 05.03.2003
(51) Int. Cl.: A61K 31/375, A61P 27/02

(54) **USE OF AN L-ASCORBIC ACID SALT TO PREPARE A PHARMACEUTICAL COMPOSITION FOR OPHTHALMIC TOPICAL USE CAPABLE OF IMPROVING THE LEVEL OF L-ASCORBIC ACID IN THE EYE**
VERWENDUNG EINES L-ASCORBINSÄURESALZES ZUR HERSTELLUNG EINER PHARMAZEUTISCHEN ZUSAMMENSETZUNG FÜR DIE OPHTHALMISCHE TOPISCHE ANWENDUNG ZUR VERBESSERUNG DES L-ASCORBINSÄURESPIEGELS IM AUGE
UTILISATION D'UN SEL D'ACIDE L-ASCORBIQUE POUR PREPARER UNE COMPOSITION PHARMACEUTIQUE POUR UNE UTILISATION TOPIQUE OPHTALMIQUE CAPABLE D'AMELIORER LE NIVEAU D'ACIDE L-ASCORBIQUE DANS L'OEIL

(30) Priority: 15.03.2002 IT MI20020557
(43) Date of publication of application: 15.12.2004
(73) Proprietor: AZIENDE CHIMICHE RIUNITE ANGELINI FRANCESCO A.C.R.A.F. S.p.A., 00181 Roma (IT)
(72) Inventor: PINZA, Mario, I-20094 Corsico (IT); DURANDO, Lucia, I-00151 Roma (IT); MARCHITTO, Leonardo, I-62017 Porto Recanati (IT); VIRNO, Michele, I-00136 Roma (IT)
(74) Representative: Marchi, Massimo
(86) International application number: PCT/EP2003/002258
(87) International publication number: WO 2003/077905

(56) References cited:
- EP-A- 0 781 547
- WO-A-02/01954
- DE-A- 3 133 003
- DE-A- 19 729 879
- US-A- 4 620 979
- US-A- 4 886 815
- US-A- 5 523 316
- US-A- 5 817 630
- PATENT ABSTRACTS OF JAPAN vol. 007, no. 095 (C-163), 23 August 1983 (1983-08-23) & JP 58 021614 A (ESUESU SEIYAKU KK), 8 February 1983 (1983-02-08)

## Description

The present invention relates to the use of a salt of L-ascorbic acid with a pharmaceutically acceptable organic base to prepare a pharmaceutical composition, for ophthalmic topical use, capable of improving the level of L-ascorbic acid in the eye. The invention also relates to a therapeutic method that comprises the topical administration of the said salt to an eye whose level of L-ascorbic acid it is desired to improve.

It is known that the levels of L-ascorbic acid in the aqueous humour (1.06 mmol/l; Arshinoff S.A., et al. "Ophthalmology", chapter 4.20.2, published by Mosby Intemational Ltd., 1999) are about 20 times higher (Brubaker R.F. et al. "Investigative Ophthalmology & Visual Science", June 2000, vol. 41, No. 7, pp. 1681) than those present in the blood circulation (20-70 µmol/l, Geigy Scientific Tables, vol. 3, page 132, 8th edition 1985, published by Ciba Geigy). In the case of the retina, the levels of L-ascorbic acid in the eye are actually 100 times higher than those present in the blood circulation.

WO 02 01954 discloses the medical use of ascorbic acid and its salts for recovering cellular function following injury. No organic salt of ascorbic acid is specifically mentioned in this document. EP-A-0 781 547 discloses an ophthalmic formulation, for use in eye surgery, comprising sodium hyaluronate, citrate, at least one antioxidant tolerated by the intraocular tissues and a phosphate buffer. The antioxidant may be an ascorbate. More specifically, the ascorbate is sodium ascorbate.

Despite the fact that the role of L-ascorbic acid in the eye has still not been definitively clarified, it is thought that its antioxidant capabilities prevent the crystallization and accumulation of foreign molecules that would interfere with the visual function.

Moreover, it has been proven that:
- the level of L-ascorbic acid reduces in an eye suffering from cataracts and glaucoma, and
- the systemic administration of L-ascorbic acid reduces the risk of cataracts and decreases the intraocular pressure in an eye suffering from glaucoma.

It is also known that a number of drugs administered ophthalmically cause the undesired effect of reducing the level of L-ascorbic acid in the eye. Typical examples of drugs that cause this undesired effect are steroidal anti-inflammatories such as dexamethasone.

The inventors have now found that the behaviour of salts of L-ascorbic acid with alkali metals is, unexpectedly, different from that of the salts with organic bases, since the comea is not permeable to the former salts, whereas it is to the latter salts. To the inventors' knowledge, the pronounced difference they found experimentally (Table I) was not at all foreseeable.

The topical ophthalmic administration of a salt of L-ascorbic acid with a pharmaceutically acceptable organic base is thus advantageous in the prevention and treatment of cataracts and glaucoma. It will moreover be advantageous to combine the administration of the said salt with that of a drug for ophthalmic use that causes a reduction in L-ascorbic acid in the eye, so as to compare the said undesired effect of the said drug.

In a first aspect, the present invention thus relates to the use of a salt of L-ascorbic acid with a pharmaceutically acceptable organic base to prepare a pharmaceutical composition, for ophthalmic topical use, capable of improving the level of L-ascorbic acid in a human eye.

In the course of the present description and in the claims, the term "improve" is used in the relative sense since, when the said salt is administered for therapeutic purposes to an eye presenting a deficiency in ascorbic acid, without this deficiency having been caused by a drug, it effectively has the purpose of increasing the level of L-ascorbic acid in the treated eye. On the other hand, when the said salt is administered to compare the undesired effect of a drug that reduces the level of L-ascorbic acid in the eye, the said salt improves the level of L-ascorbic acid relative to the level that would be reached if the drug was administered alone. Achieving a level intermediate between the higher initial level (basal) and the lower level caused by the administration of a drug having the abovementioned undesired effect thus also forms part of the invention.

Advantageously, the said organic base is chosen from the group comprising tromethamine, N-methylglucosamine, lysine, arginine and omithine.

Tromethamine and lysine are particularly preferred.

The pharmaceutical composition according to the present invention is preferably prepared in suitable dosage forms comprising an effective dose of at least one salt of L-ascorbic acid with a pharmaceutically acceptable organic base and at least one pharmaceutically acceptable inert vehicle.

Examples of suitable dosage forms are creams and sterile solutions. These solutions may be ready-to-use or may be prepared at the time of use by dissolving a sterile powder or lyophilizate in a pharmaceutically acceptable sterile liquid vehicle.

The dosage forms may also contain other conventional ingredients such as: preserving agents, stabilizers, surfactants, dispersants, salts for regulating the osmotic pressure, emulsifiers, colorants and the like.

The amount of the said salt of L-ascorbic acid with a pharmaceutically acceptable organic base in the said pharmaceutically acceptable inert vehicle is typically between 0.1 and 20 mg/ml. Preferably, the said amount is between 0.2 and 10 mg/ml and even more preferably between 0.5 and 2 mg/ml.

The administration of the salt according to the present invention does not involve any undesired effects and may be carried out several times a day. Typically, the said administration is carried out 1 to 24 times a day and preferably from 3 to 12 times a day. However, in the case of compositions also containing another drug, the specific therapeutic regime of the said drug Will be followed.

The dosage forms of the pharmaceutical composition of the present invention may be prepared according to techniques, that are well known in pharmaceutical chemistry, including mixing, lyophilization, dissolution, sterilization and the like.

When a salt of L-ascorbic acid with a pharmaceutically acceptable organic base is administered to a patient to compare the reduction in the level of L-ascorbic acid in the eye caused by the administration of another drug, a single dosage form comprising both the said salt and the said drug may advantageously be envisaged.

A typical example of this dosage form is a collyrium comprising an anti-inflammatory drug such as dexamethasone and a salt of L-ascorbic acid with a pharmaceutically acceptable organic base according to the present invention.

When these unit dosage forms, which comprise both a salt of L-ascorbic acid with a pharmaceutically acceptable organic base and a drug capable of reducing the level of L-ascorbic acid in the eye, are In the form of sterile aqueous solutions, the salt according to the present invention may advantageously also act as a buffer.

As mentioned previously, the administration of the salt according to the present invention does not involve any undesired effects and may be carried out several times a day. Typically, the therapeutic method of the present invention comprises from 1 to 24 administrations and preferably from 3 to 12 administrations a day of a pharmaceutical form comprising from 0.1 to 20 mg/ml of the said salt of L-ascorbic acid with a pharmaceutically acceptable organic base. Preferably, the said pharmaceutical form comprises from 0.2 to 10 mg/ml and even more preferably from 0.5 to 2 mg/ml of the said salt of L-ascorbic acid with a pharmaceutically acceptable organic base.

However, when the salt according to the present invention is administered in combination with another drug, the specific therapeutic regime of the said drug will be followed.

The following examples serve to illustrate the invention without, however, limiting it.

### EXPERIMENTAL SELECTION

| Composition A | |
|---|---|
| Lysine ascorbate | 0.909 g |
| Natrosol 250 | 1.200 g |
| NaCl | 0.350 g |
| Distilled water qs | 100 ml |
| pH | 5.3-5.6 |

| Composition B | |
|---|---|
| Tromethamine ascorbate | 0.838 g |
| Natrosol 250 | 1.200 g |
| Distilled water qs | 100 ml |
| pH | 5.0-5.6 |

### Comparative Composition 1

The collyrium Luxazone™ sold in Italy by the company Allorgan was used. Said collyrium consists of an aqueous 0.2% solution. The other ingredients are monosodium phosphate, sodium sulphite heptahydrate, sodium chloride, benzalkonium chloride, hydroxypropylcellulose and polysorbate 80.

### Comparative composition 2

| | |
|---|---|
| Sodium ascorbate | 0.562 g |
| Natrosol 250 | 1.200 g |
| KH₂PO₄ | 1.000 g |
| K₂HPO₄ | 0.270 g |
| NaCl | 0.250 g |
| Distilled water qs | 100ml |
| pH | 5.0-5.5 |

### TEST 1

### Transcomeal passage of L-ascorbic acid

### 1. Aim of the experiment

The purpose of the test was to confirm the passage of L-ascorbic acid across the cornea under experimental conditions of a reduced level of endogenous L-ascorbic acid in the aqueous humour.

The experimental model used involved measuring the level of L-ascorbic acid in the aqueous humour first under basal conditions and then after having induced a reduction in the level of endogenous L-ascorbic acid in the aqueous humour by means of chronic treatment with Comparative Composition 1 of the prior art.

The effect on the level of endogenous L-ascorbic acid of Comparative Composition 2 and of Compositions A and B according to the invention was also evaluated in the same way.

### 2. Experimental model

Male New Zealand White rabbits (2.5-4 kg) were used.

The animals, divided into groups of 3 animals each, were treated according to the following scheme:

| | |
|---|---|
| Group A1 | : Comparative Composition 1 |
| Group A2 | : Comparative Composition 1 |
| | + Comparative Composition 2 |
| Group B1 | : Comparative Composition 1 |
| Group B2 | : Comparative Composition 1 |
| | + Composition A of the invention |
| Group C1 | : Comparative Composition 1 |
| Group C2 | : Comparative Composition 1 |
| | + Composition B of the invention |

The animals were treated topically (100 µl/eye) in both eyes 3 times a day for about 2 weeks.

The measurement of the level of endogenous L-ascorbic acid in the aqueous humour was carried out before the start of the treatment (basal value) and 2 weeks after the start of the treatment (14th day).

The level of endogenous L-ascorbic acid was measured in aqueous humour removed by paracentesis, under general and topical anaesthesia, from the right and left eye alternately.

The determination of the L-ascorbic acid was carried out using a Merck RQflex® plus reflectometer, working according to the Merck test 1.16981.0001 which is based on the property of ascorbic acid to reduce yellow molybdophosphoric acid to blue phosphomolybdenum and reflectometric measurement of the latter.

### 3. Results

The levels of L-ascorbic acid (mg/l) in the aqueous humour of rabbits measured on day 0 and on day 14 are shown in the following table, in which the value in parentheses indicates the change relative to the basal reading.

**Table I**

| Group | Day 0 | Day 14 |
|---|---|---|
| A1 | 211 | 68 (-68%) |
| A2 | 197 | 63 (-68%) |
| B1 | 192 | 49 (-74%) |
| B2 | 198 | 78 (-61%) |
| C1 | 149 | 69 (-53%) |
| C2 | 162 | 101 (-38%) |

The abovementioned results show that:
- in Group A1, a pronounced reduction (68%) in the level of endogenous L-ascorbic acid was measured after 14 days of treatment with dexamethasone alone (Comparative Composition 1);
- in Group A2, this reduction was not affected by simultaneous administration of a salt of ascorbic acid with a mineral base (sodium ascorbate: Comparative Composition 2);
- in Group B1, the reduction in the level of endogenous L-ascorbic acid after 14 days of treatment with dexamethasone alone (Comparative Composition 1) was 74%;
- in Group B2, this reduction was affected by simultaneous administration of a salt of ascorbic acid with an organic base (lysine ascorbate: Composition A). Specifically, in this case, the reduction in the level of endogenous L-ascorbic acid after 14 days of treatment was 61% rather than 74%;
- in Group C1, the reduction in the level of endogenous L-ascorbic acid after 14 days of treatment with dexamethasone alone (Comparative Composition 1) was 53%;
- in Group C2, this reduction was effectively affected by with the simultaneous administration of a salt of ascorbic acid with an organic base (tromethamine ascorbate: Composition B). Specifically, the reduction in the level of endogenous L-ascorbic acid after 14 days of treatment was only 38%, rather than 53%.

The abovementioned results therefore prove that, unlike the salts with mineral bases, the salts of ascorbic acid with organic bases pass through the cornea. This effect was particularly pronounced in the case of the tromethamine salt.

## Claims

1. Use of a salt of L-ascorbic acid with a pharmaceutically acceptable organic base to prepare a pharmaceutical composition, for ophthalmic topical use, capable of improving the level of L-ascorbic acid in a human eye.

2. Use according to Claim 1, **characterized in that** the said organic base is chosen from the group comprising tromethamine, N-methylglucosamine, lysine, arginine and omithine.

3. Use according to Claim 1, **characterized in that** the said organic base is tromethamine or lysine.

4. Use according to any one of Claims 1 to 3, **characterized in that** the said composition is a cream or a sterile solution.

5. Use according to any one of Claims 1 to 4, **characterized in that** the said composition comprises from 0.1 to 20 mg/ml of the said salt of L-ascorbic acid with a pharmaceutically acceptable organic base and at least one pharmaceutically acceptable inert vehicle.

6. Use according to Claim 5, **characterized in that** the said composition comprises from 0.2 to 10 mg/ml of the said salt of L-ascorbic acid with a pharmaceutically acceptable organic base and at least one pharmaceutically acceptable inert vehicle.

7. Use according to Claim 5, **characterized in that** the said composition comprises from 0.5 to 2 mg/ml of the said salt of L-ascorbic acid with a pharmaceutically acceptable organic base and at least one pharmaceutically acceptable inert vehicle.

8. Use according to any one of Claims 1 to 7, **characterized in that** the said composition is a sterile collyrium comprising a salt of L-ascorbic acid with lysine or with tromethamine.

9. Use according to Claim 8, **characterized in that** the said composition also comprises an anti-inflammatory drug.

10. Use according to Claim 9, **characterized in that** the said

## Patentansprüche

1. Verwendung eines Salzes von L-Ascorbinsäure mit einer pharmazeutisch annehmbaren organischen Base zur Herstellung einer pharmazeutischen Zusammensetzung zur topischen ophthalmologischen Anwendung, die zur Verbesserung des L-Ascorbinsäurenniveaus im menschlichen Auge in der Lage ist.

2. Verwendung gemäß Anspruch 1, **dadurch gekennzeichnet, dass** die organische Base ausgewählt ist aus Tromethamin, N-Methyl-Glucosamin, Lysin, Arginin und Ornithin.

3. Verwendung gemäß Anspruch 1, **dadurch gekennzeichnet, dass** die organische Base Thromethamin oder Lysin ist.

4. Verwendung gemäß mindestens einem der Ansprüche 1-3, **dadurch gekennzeichnet, dass** die Zusammensetzung eine Creme oder eine sterile Lösung ist.

5. Verwendung gemäß mindestens einem der Ansprüche 1-4, **dadurch gekennzeichnet, dass** die Zusammensetzung 0,1-20 mg/ml des Salzes von L-Ascorbinsäure mit einer pharmazeutisch annehmbaren organischen Base und mindestens einen pharmazeutisch annehmbaren inerten Träger umfasst.

6. Verwendung gemäß Anspruch 5, **dadurch gekennzeichnet, dass** die Zusammensetzung 0,2-10 mg/ml des Salzes von L-Ascorbinsäure mit einer pharmazeutisch annehmbaren organischen Base und mindestens einen pharmazeutisch annehmbaren inerten Träger umfasst.

7. Verwendung gemäß Anspruch 5, **dadurch gekennzeichnet, dass** die Zusammensetzung 0,5-2 mg/ml des Salzes von L-Ascorbinsäure mit einer pharmazeutisch annehmbaren organischen Base und mindestens einen pharmazeutisch annehmbaren inerten Träger umfasst.

8. Verwendung gemäß mindestens einem der Ansprüche 1-7, **dadurch gekennzeichnet, dass** die Zusammensetzung ein steriles Collyrium ist, das ein Salz von L-Ascorbinsäure mit Lysin oder Thromethamin umfasst.

9. Verwendung gemäß Anspruch 8, **dadurch gekennzeichnet, dass** die Zusammensetzung ferner einen entzündungshemmenden Wirkstoff umfasst.

10. Verwendung gemäß Anspruch 9, **dadurch gekennzeichnet, dass** der entzündungshemmende Wirkstoff Dexamethason ist.

## Revendications

1. Utilisation d'un sel d'acide L-ascorbique avec une base organique pharmaceutiquement acceptable pour préparer une composition pharmaceutique, pour une utilisation ophtalmique topique, capable d'augmenter le taux d'acide L-ascorbique dans un oeil humain.

2. Utilisation selon la revendication 1, **caractérisée en ce que** ladite base organique est choisie dans le groupe constitué par la trométhamine, la N-méthylglucosamine, la lysine, l'arginine et l'ornithine.

3. Utilisation selon la revendication 1, **caractérisée en ce que** ladite base organique est la trométhamine ou la lysine.

4. Utilisation selon l'une quelconque des revendications 1 à 3, **caractérisée en ce que** ladite composition est une crème ou une solution stérile.

5. Utilisation selon l'une quelconque des revendications 1 à 4, **caractérisée en ce que** ladite composition comprend de 0,1 à 20 mg/ml dudit sel d'acide L-ascorbique avec une base organique pharmaceutiquement acceptable et au moins un véhicule inerte pharmaceutiquement acceptable.

6. Utilisation selon la revendication 5, **caractérisée en ce que** ladite composition comprend de 0,2 à 10 mg/ml dudit sel d'acide L-ascorbique avec une base organique pharmaceutiquement acceptable et au moins un véhicule inerte pharmaceutiquement acceptable.

7. Utilisation selon la revendication 5, **caractérisée en ce que** ladite composition comprend de 0,5 à 2 mg/ml dudit sel d'acide L-ascorbique avec une base organique pharmàceutiquement acceptable et au moins un véhicule inerte pharmaceutiquement acceptable.

8. Utilisation selon l'une quelconque des revendications 1 à 7, **caractérisée en ce que** ladite composition est un collyre stérile comprenant un sel d'acide L-ascorbique avec la lysine ou avec la trométhamine.

9. Utilisation selon la revendication 8, **caractérisée en ce que** ladite composition comprend également un médicament antiinflammatoire.

10. Utilisation selon la revendication 9, **caractérisée en ce que** ledit médicament antiinflammatoire est la dexaméthasone.
